(19) Europäisches Patentamt
European Patent Office
Office européen des brevets

(11) Publication number: **0 037 742**
**B1**

(12) **EUROPEAN PATENT SPECIFICATION**

(45) Date of publication of patent specification: **19.12.84**

(51) Int. Cl.$^3$: **C 12 Q 1/32,** G 01 N 33/74

(21) Application number: **81301538.5**

(22) Date of filing: **08.04.81**

(54) **Method for the estimation of hydroxysteroids and reagent system therefor.**

(30) Priority: **09.04.80 GB 8011730**

(43) Date of publication of application:
**14.10.81 Bulletin 81/41**

(45) Publication of the grant of the patent:
**19.12.84 Bulletin 84/51**

(84) Designated Contracting States:
**AT BE CH DE FR GB IT LI LU NL SE**

(56) References cited:
**GB-A-1 397 406**

**CLINICAL CHEMISTRY, Vol. 26, No. 9, August 1980 M.J. CROWELL et al. "Enzymic Determination of 3Alpha - 7Alpha - and 12Alpha-Hydroxyl Groups of Fecal Bile Salts" pages 1298 to 1300**

**The file contains technical information submitted after the application was filed and not included in this specification**

(73) Proprietor: **NYEGAARD & CO. A/S**
**Nycoveien 2 Postboks 4220**
**Oslo 4 (NO)**

(72) Inventor: **Yamanaka, Manabu**
**Bunkyo-ku**
**Tokyo (JP)**
Inventor: **Mashige, Fumiko**
**No 1-6-707, 2-chome**
**Kasuga, Bunkyo-ku Tokyo (JP)**
Inventor: **Matsumura, Yoshinori**
**No 22-21-506, 5-chome**
**Niijyuka, Katsushika-ku Tokyo (JP)**
Inventor: **Skalhegg, Bjorn Arne**
**Hagaliveien 2**
**1342 Jar, Baerum (NO)**

(74) Representative: **Holmes, Michael John et al**
**Frank B. Dehn & Co. European Patent Attorneys**
**Imperial House 15-19 Kingsway**
**London, WC2B 6UZ, (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 037 742 B1

## Description

This invention concerns a novel method and reagent system for the estimation of hydroxysteroids.

It has been proposed to estimate hydroxysteroids, for example, in human serum, by enzymic dehydrogenation in the presence of a coenzyme such as nicotinamide adenine dinucleotide (NAD) [see British Patent Specification No. 1,397,406]. The NAD is hydrogenated during the reaction to the dihydro-form (NADH) and the extent of the reaction is measured by measuring the extinction coefficient of NAD at 340 nm i.e. using ultraviolet light. However, such measurements require relatively costly equipment and it would be much more convenient if measurements could be made using visible light, i.e. using a colorimeter.

It has been found that the above reaction, involving conversion of NAD to NADH can be coupled with a further enzymic reaction, namely the hydrogenation of a tetrazolium salt in the presence of diaphorase to yield a formazane dye, which can be measured with acceptable sensitivity at 540 nm i.e. using visible light. This reaction system can be represented by the following scheme:—

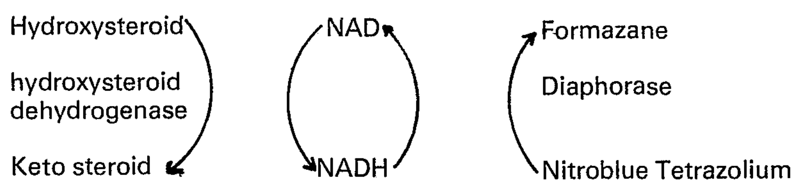


Serum normally contains enzymes such as lactate dehydrogenase (LDH) which can also react with the reagent system and consequently distort the estimation. We have found, however, that such competing enzymes can be deactivated substantially completely by addition of a non-steroidal hydroxyacid dehydrogenase inhibitor such as oxamic or pyruvic acid or salts thereof, to the serum without significantly affecting the steroid dehydrogenase or diaphorase enzymes used in the test and that this inhibitor can, surprisingly be added to the serum simultaneously with the test reagent or, more conveniently, can be made a component of the test reagent.

A test reagent based on this principle can thus comprise a hydroxysteroid dehydrogenase (HSD), a coenzyme therefor, diaphorase and a tetrazolium salt together with an inhibitor of non-steroidal hydroxyacid dehydrogenase. Such a reagent should naturally be free from hydroxysteroids and other serum components which might interfere with the test and this provides a distinction from any previous combination of the above components which may have existed transiently during a serum analysis.

According to the present invention, therefore, we provide a reagent for the estimation of hydroxysteroids in serum comprising a hydroxysteroid dehydrogenase, a coenzyme therefor, diaphorase, a tetrazolium salt and an inhibitor of non-steroidal hydroxyacid dehydrogenases, the said reagent being substantially free from hydroxysteroids and other serum components.

According to a further feature of the invention we provide a method for the estimation of hydroxy acids in serum, whereby said hydroxyacids are oxidised to keto acids by substantially simultaneous addition to the serum or diluted serum of a hydroxysteroid dehydrogenase, a coenzyme therefor, a diaphorase, a tetrazolium salt and an inhibitor of non-steroidal hydroxyacid dehydrogenases, whereby a formazane dye is formed and is estimated colorimetrically.

The new reagent and method are particularly useful for the estimation of hydroxysteroids in human serum in studying steroid metabolism, especially in adrenocortical and gonadal function and particularly in the study of bile acids and conjugates thereof, which can often be of assistance in the diagnosis of liver diseases. Such bile acids include, for example, deoxycholic, lithocholic and chenodeoxycholic acids. These are all $3\alpha$-hydroxyacids and in the estimation of these, the hydroxysteroid dehydrogenase will normally be a specific $3\alpha$-hydroxyacid dehydrogenase (for example as sold by Nyegaard & Co. A/S under the name Sterognost $3\alpha$ [Trade Mark]).

However, the primary bile acids possess a $7\alpha$-hydroxyl group in addition to the $3\alpha$-hydroxy group and bacterial $7\alpha$-dehydroxylation may take place in the intestine and colon to form secondary bile acids lacking the $7\alpha$-hydroxyl group. Thus, cholic acid ($3\alpha$, $7\alpha$, $12\alpha$-trihydroxy-cholanic acid) produces deoxycholic acid and ($3\alpha$, $7\alpha$-dihydroxycholanic acid) produces lithocholic acid ($3\alpha$-hydroxycholanic acid). The ratio of primary to secondary bile acids may vary from disease to disease, for example being reduced when the intestine is infected with undesirable bacteria and the synthetic capacity of the liver with respect to the primary bile acids may also be affected by liver diseases. It is thus of great interest to estimate total $7\alpha$-hydroxysteroids i.e. total primary bile acids independently from total $3\alpha$-hydroxysteroids, substraction giving the value for total secondary bile acids. A suitable $7\alpha$-HSE enzyme is sold by Nyegaard & Co. A/S under the name Sterognost $7\alpha$ [Trade Mark].

The reagent system according to the invention is preferably in solid form to facilitate storage, although it may, if desired, be made up from the individual components shortly before use. The reagent is conveniently in lyophilised form. Such solid forms are preferably dissolved in water or buffer to form

an aqueous reagent system prior to use. A buffer such as tris hydrochloride will commonly be present to maintain a pH in the test system of about 8.0. In general, the pH of the test system may be in the range 7.0 to 8.5.

The tetrazolium salt will generally be of the formula

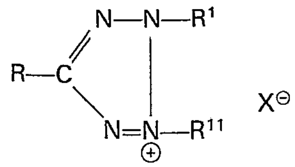

where $X^{\ominus}$ is an anion, such as halide, e.g. chloride or bromide, R is a hydrocarbon group and $R^1$ and $R^{11}$ are both aromatic cyclic groups, the groups, R, $R^1$ and $R^{11}$ optionally carrying substituents. In general R may be $C_{1-12}$ alkyl or phenyl, optionally carrying one or more substituents such as halo, nitro or $C_{1-5}$ alkoxycarbonyl; $R^1$ and $R^{11}$, which may be the same or different, may be, for example phenyl or thiazolyl rings, which may be substituted, e.g. by nitro and/or $C_{1-5}$ alkoxy groups. In particular, one group $R^1$ or $R^{11}$ may carry as a substituent a further phenyl ring carrying a tetrazolium grouping so as to form a symmetrical dimer. Nitroblue tetrazolium (NTB) is of this latter type, being 3,3′(3,3′-dimethoxy-4,4′-diphenylene)bis[2-(*p*-nitrophenyl)-5-phenyl-tetrazolium chloride]. Other very useful tetrazolium salts include INT (2-(*p*-nitrophenyl)-3-(*p*-iodophenyl)-5-phenyltetrazolium chloride) and 4,5-MTT (2-[4′,5′-dimethylthiazolyl-(2′)]-3,5-diphenyltetrazolium bromide).

The coenzyme is preferably NAD but may be an equivalent compound such as thionicotinamide adenine dinucleotide (TNAD).

As mentioned above, for use the reagent is preferably in the form of an aqueous solution. The HSD may be present in the aqueous reagent system in the concentration range 0.25 IU to 3.0 IU, preferably 0.5 to 1.5 IU per 100 ml reagent. The coenzyme, such as NAD, may be present in the aqueous reagent system in the concentration range 25—600 μmol, preferably 50 to 400 μmol per 100 ml reagent. The diaphorase may be present in the concentration range 12 to 150 IU, preferably 25 to 75 IU per 100 ml reagent. The concentration of the tetrazolium salt (particularly NTB) is rather critical, in that the aqueous solubility of both the salt and the formazane eventually formed, is rather limited. In general, the concentration of tetrazolium salt (TS) in the aqueous reagent system may be in the range 3 to 300 μmol. At a level of 3 μmol TS per 100 ml reagent, it is only possible to determine accurately concentrations of hydroxysteroids at up to about 75 μmol per litre, since the standard curves then become non-linear. In order to obtain linear standard curves at relatively high concentrations of hydroxysteroids, such as are observed in some pathological cases, it is desirable to use much higher concentrations of TS. A preferred range is 50 to 250 μmol per 100 ml reagent; where however, the reagent is made up from a lyophilised mixture, as is described in detail hereinafter, the concentration of TS must be kept below 180 μmol/100 ml reagent, particularly where NTB is used.

In general, the concentration of the inhibitor of non-steroidal hydroxyacid dehydrogenases in the aqueous test reagent will be in the range 0.05 to 8.0 g per 100 ml. Preferred inhibitors are oxamic or pyruvic acid or a salt thereof such as for example an alkali metal salt e.g. the sodium salt. The concentration of oxamic acid or salt thereof in the aqueous test reagent system may be in the range 0.05 to 0.8 g, preferably 0.2 to 0.4 g per 100 ml reagent. The concentration of pyruvic acid or salt thereof in the final aqueous test reagent system may be in the range 2 to 8 g per 100 ml reagent e.g. about 5.0 g.

In general, the estimation of the sample will be accompanied by simultaneous estimations of purified water, a standard serum (S1) and a standard serum containing a known quantity of an appropriate hydroxysteroid (S2). Each of these will be reacted with the above reagent system and, in parallel, with a blank reagent identical with the principal reagent but omitting the steroid dehydrogenase.

According to a further feature of the invention we provide a reagent for the estimation of hydroxysteroids in serum in conjunction with a reagent as previously defined according to the invention comprising a coenzyme for a hydroxysteroid dehydrogenase, diaphorase, a tetrazolium salt and an inhibitor of non-steroidal hydroxyacid dehydrogenases, the said reagent being substantially free from hydroxysteroid dehydrogenases, hydroxysteroids and other serum components.

The solutions are incubated, for example at about room temperature e.g. 23°C, or higher e.g. about 37°C; the incubation time will vary with temperature, e.g. about 15—20 minutes at 23°C or about 10 minutes at 37°C.

The two series of serum-containing solutions (blank reagent and normal reagent) are each then estimated colorimetrically, preferably at about 540 nm against the corresponding solutions prepared from purified water to give corrected optical density values (OD's). The differences (ΔOD's) between the blank reagent and normal reagent solutions in respect of the sample and the two standards are then calculated and the concentrations of hydroxysteroid in the serum is given by the equation:—

$$\text{Total hydroxysteroid} \frac{(\mu mol/ml) = X \times \Delta OD \text{ of serum sample}}{(\text{Difference between } \Delta OD\text{'s of standards})}$$

X being the concentration of hydroxysteroid in standard S2 in $\mu$mol/ml.

It may be preferable to stop the enzymic reaction rapidly at the end of the predetermined time, for example by addition of acid, e.g. 2N hydrochloric or 4N phosphoric acid. It may be advantageous to add a wetting agent, to assist in preventing the formazane from precipitating, particularly when high concentrations of TS are used. However, if the colorimetric estimations are carried out rapidly, it may not be necessary to stop the reaction in this way, since only a very slow increase of OD is normally observed at the end of the incubation time. Similarly, if relatively low concentrations of NTB are used, no wetting agent is required. It may be advantageous to include the wetting agent in the enzyme reagent, provided it is compatible. In general, the wetting agent or surfactant will be of the non-ionic type. Suitable surfactants include polyoxyethylene derivatives of long chain alcohols (e.g. $C_{10-20}$ alcohols such as cetyl, lauryl, stearyl and oleyl alcohols); polyoxyethylene derivatives of alkylphenols, (e.g. $C_{7-10}$ alkyl-substituted phenols such as isooctyl or nonyl phenols), polyoxyethylene derivatives of sugar alcohol esters of fatty acids (e.g. mannitan or sorbitan esters of $C_{10-20}$ fatty acids such as oleic or stearic acid) and polyoxyethylene esters of alkylphosphoric acids. In general, the surfactants will have 9 to 40 oxyethylene units per molecule, the HLB values advantageously being in the range 11—20. Suitable surfactants include Emalugen 120, 147, 409P, 909 and 913, Noigen 400 and ET 180, Bij 35, Liponox NCI and NCK, Triton X-100, Plysurf A215-C and A219-B and Tween 80 [The words "Emalugen", "Noigen", "Bij", "Liponox", "Triton", "Plysurf", and "Tween" are Trade Marks]. While slight inhibition of the reagent system is observed with some surfactants, Noigen ET 180 had the least inhibitory effect, being significantly better than Triton X-100.

Since it is important that the above techniques be rigorously standardised and, at the same time, easily and rapidly performed, it is particularly useful to make up the reagents from pre-prepared lyophilised mixtures which merely have to be re-dissolved in purified water immediately before use.

According to a particularly important feature of the invention, therefore, we provide lyophilised mixtures containing a steroid dehydrogenase, a coenzyme therefor, diaphorase, a tetrazolium salt and an inhibitor of non-steroidal hydroxyacid dehydrogenases. We also provide lyophilised mixtures of said coenzyme, diaphorase, tetrazolium salt and inhibitor for use as the "blank reagent" in the above test system.

However, we have found considerable difficulty in formulating a reagent system capable of being lyophilised and redissolved in a rigorously standardised manner. The principal problem is the relatively low solubility of the tetrazolium salt (e.g. NTB) and formazane in the aqueous media used. Although it may be possible to use TS at over 200 $\mu$mol per 100 ml reagent when simply dissolving the separate components of the reagent, when lyophilising it is essential that the concentration does not exceed 180 $\mu$mol/100 ml reagent, since otherwise the TS and in particular NTB tends to become irreversibly insoluble after lyophilisation and during incubation, leading to unsatisfactory results. In general, we have found it difficult to incorporate surfactants into the lyophilised reagent to counter this problem and in any case this expedient does not overcome the above irreversible loss of solubility. Optimal results are obtained in the range 40 to 155 $\mu$mol TS per 100 ml reagent. The lowest concentration of TS which gives adequate results in most cases is about 3 $\mu$mol per 100 ml reagent. When using these concentrations of TS, the concentrations of other components in the reagent system will be at appropriate levels to ensure that the hydroxysteroid is fully dehydrogenated.

We have further found that the system can be additionally stabilised by incorporation of albumin. For optimal results, the concentration of albumin in the reagent should preferably be in the range 40 to 100 mg, advantageously about 70 mg albumin per 100 ml reagent. Higher concentrations may have an adverse effect on the sensitivity of the system. Bovine serum albumin is one convenient type of albumin.

It is also particularly beneficial to include in the lyophilised reagent, i.e. in the solution of the reagent prior to lyophilisation, a non-reducing polyol such as a sugar alcohol, e.g. mannitol or sorbitol, a non-reducing sugar such as sucrose or a non-reducing polysaccharide such as dextran. The concentration of mannitol in the reagent is advantageously in the range 0.1 to 16 g/100 ml, e.g. 1 to 6 g/100 ml. The concentration of dextran in the reagent may be in the range 0.1 to 30 g/100 ml, advantageously 0.5 to 1.5 g/100 ml.

The incorporation of an inhibitor such as oxamic or pyruvic acid into the solutions for lyophilisation not only simplifies the procedure in eliminating a separate reaction stage but also appears to assist in stabilising the reagent and increasing the sensitivity. In particular, oxamic or pyruvic acid appears to permit relatively high concentrations of TS to be lyophilised without becoming irreversibly insoluble. Furthermore, oxamic or pyruvic acid also enables relatively high concentrations of albumin to be used without adversely affecting sensitivity, thereby permitting even greater stability to be achieved.

The invention also included a diagnostic reagent kit comprising a reagent according to the invention containing HSD and a blank reagent according to the invention omitting HSD, both reagents preferably being in lyophilised form.

The following Examples are given by way of illustration only:—

Example 1

Reagents

(1) The following components were dissolved in purified water:

| | |
|---|---|
| 3α-hydroxysteroid dehydrogenase (Nyegaard & Co. A/S: Sterognost 3α) | 1 IU/140 ml reagent |
| NAD | 200 μmol/140 ml reagent |
| Diaphorase | 50 IU/140 ml reagent |
| NTB | 61 μmol/140 ml reagent |
| Oxamic acid | 0.29% |
| Buffer:0.2N Tris Hydrochloride | (pH 9.0) to 140 ml. |

This reagent mixture was lyophilised and then redissolved in 140 ml purified water.

(2) The same reagent as reagent (1), but without 3α-HSD

(3) 2N HCl containing 10% Triton X-100

(4) standard: St-1=pooled serum, St-2=pooled serum added 50 mol/l of glycocholic acid

Procedure

a) Pipette two samples each of the serum sample, 0.2 ml of purified water, serum sample, St-1 and St-2 into separate brown test tubes;

b) for each of the four pairs of test tubes, add 0.7 ml of reagent (1) to one test tube and add 0.7 ml of reagent (2) to the other test tube, and incubate at 37°C accurately for 10 min;

c) immediately after the incubation, stop the reaction by the addition of 2N HCl containing 10% Triton X-100 to each test tube;

d) determine the absorptivity of reaction mixture of each test tube containing reagent (1), and each test tube containing reagent (2) against corresponding test tube of purified water using a colorimeter cell of 1 ml volume at 540 nm; and;

e) determine the difference of absorptivity in each pair of the test tubes (ΔOD)

Calculation

Total bile acid (μmol/ml)=(50×ΔOD of serum sample)/(ΔOD of st-2 minus ΔOD of st-1)

Essentially similar results are obtained when the NTB is replaced by an equal quantity of INT or 4,5-MTT.

Example 2

The procedure and reagent system of Example 1 were used with incubation at 23°C instead of 37°C. The optimal incubation time was found to be about 15—20 minutes.

Example 3

The procedure and reagent system of Example 1 was used with addition to reagents (1) and (2) of 100 mg bovine serum albumin per 140 ml reagent solution.

Example 4
Reagents
Reagent (1)
In 1 l of 0.1 *M* Tris-1, pH 9.0, dissolve the following reagents:

| | |
|---|---|
| 3α-HSD (Nyegaard Co. Oslo) | 10 I.U. |
| Diaphorase | 500 units |
| NAD | 1.3 g |
| NTB | 2.0 g |
| Nonionic surfactant | 20 g |
| Pyruvic acid | 45 g |

Reagent (2)
Same as Reagent (1) excluding 3α-HSD. The procedure of Example 1 was followed, using 4N phosphoric acid in place of 2N hydrochloric acid

Assay procedure

| | sample reagent | blank reagent |
|---|---|---|
| Human serum | 0.2 ml | 0.2 ml |
| Reagent-I-a | 0.7 | — |
| Reagent-I-b | — | 0.7 ml |
| | 37°C, 10 minutes | |
| 4 N $H_3PO_4$ | 0.1 ml | 0.1 ml |

Read the absorbance of sample reagent at 540 nm against blank reagent.
The results obtained were essentially similar to those obtained in Example 1.

Example 5
1) 357 mg NTB are dissolved in 300 ml 0.2M Tris-HCl buffer pH=9.0. (The same buffer is used throughout).
2) In a separate flask diaphorase (250 I.U.) is dissolved in 100 ml buffer.
3) 500 mg bovine serum albumin are dissolved in 100 ml buffer in another flask.
4) 1000 m mole NAD are dissolved in the albumin solution (3) which is then combined with the solution consisting of (1) mixed with (2).
5) 2.0 g. oxamic acid are dissolved in 200 ml distilled water with mild heating. The solution is cooled before being added to the solution (4).
6) The total solution is filtered once through a Watman 3 mm filter.
7) The solution 6) is divided into 2 350 ml volumes (A+B).

Part A: 3.5 I.U. 3α HSD enzyme in buffer are added. The volume of the enzyme solution is about 1/1000 of the total volume of reagent A.
Part B: A volume of buffer equal to that of the enzyme solution added to Part A is added to Part B.

The solutions are filled into brown bottles. Only 25% of the total volume should be occupied by the solution (e.g. not more than 7—8 ml in a 20 ml bottle). The flasks are set at —20°C for at least twenty-four hours. The cold flasks are placed on the pre-cooled plates in the freeze drier.

When the flask contents are dry, the stoppers are pressed on under low pressure in the vacuum chamber during which process the flasks are filled with dry $N_2$ gas.

Example 6
The procedure and reagent system of Example 5 was used; with addition to solutions A and B of 15 g mannitol per 350 ml. volume.

Example 7
The procedure and reagent system of Example 5 was used, with the addition to solutions A and B of either 3.7 g dextran per 350 ml volume (when the reagent is subsequently to be incubated at 37°C) or 10 g dextran per 350 ml volume (when the reagent is subsequently to be incubated at 23°C).

Example 8

The procedure and reagent system of Example 5 was used with addition to solutions A and B of 7.5 g mannitol and 2.5 g dextran per 350 ml volume.

The procedure and reagent system of Examples 5 to 8 can also be effected using only 560 ml total buffer volume; this increases the concentration of mannitol and/or dextran in the lyophilisation step. In all cases, the reagent is made up to 700 ml before use.

**Claims**

1. A reagent for the estimation of hydroxysteroids in serum comprising a hydroxysteroid dehydrogenase, a coenzyme therefor, diaphorase, a tetrazolium salt and an inhibitor of non-steroidal hydroxyacid dehydrogenases, the said reagent being substantially free from hydroxysteroids and other serum components.

2. A reagent for the estimation of hydroxysteroids in serum in conjunction with a reagent as claimed in claim 1 comprising a coenzyme for a hydroxysteroid dehydrogenase, diaphorase a tetrazolium salt and an inhibitor of non-steroidal hydroxyacid dehydrogenases, the said reagent being substantially free from hydroxysteroid dehydrogenases, hydroxysteroids and other serum components.

3. A reagent as claimed in claim 1 or claim 2 in solid form.

4. A reagent as claimed in claim 3 in lyophilised form.

5. A reagent as claimed in any preceding claim wherein the inhibitor is oxamic or pyruvic acid or a salt thereof.

6. A reagent as claimed in any preceding claim wherein the tetrazolium salt is nitroblue tetrazolium.

7. A reagent as claimed in any preceding claim further comprising albumin.

8. A reagent as claimed in any preceding claim containing a non-reducing polyol.

9. A diagnostic reagent kit comprising a reagent as claimed in claim 1 and a reagent as claimed in claim 2 as a blank reagent, both reagents being in lyophilised form.

10. A kit as claimed in claim 9 further comprising standard samples of bile acids in human serum.

**Patentansprüche**

1. Ein Reagens zur Bestimmung von Hydroxysteroiden im Serum, umfassend eine Hydroxysteroid-Dehydrogenase, ein Co-Enzym dafür, Diaphorase, ein Tetrazoliumsalz und einen Inhibitor von nicht-steroiden Hydroxysäure-Dehydrogenasen, wobei dieses Reagens im wesentlichen frei von Hydroxysteroiden und anderen Serumbestandteilen ist.

2. Ein Reagens zur Bestimmung von Hydroxysteroiden im Serum in Verbindung mit einem Reagens gemäß Anspruch 1, umfassend ein Co-Enzym für eine Hydroxysteroid-Dehydrogenase, Diaphorase, ein Tetrazoliumsalz und einen Inhibitor von nicht-steroiden Hydroxysäure-Dehydrogenasen, wobei dieses Reagens im wesentlichen frei von Hydroxysteroid-Dehydrogenasen, Hydroxysteroiden und anderen Serumkomponenten ist.

3. Ein Reagens, wie in Anspruch 1 oder Anspruch 2 beansprucht, in fester Form.

4. Ein Reagens, wie in Anspruch 3 beansprucht, in lyophilisierter Form.

5. Ein Reagens, wie in einem der vorhergehenden Ansprüche beansprucht, worin der Inhibitor Oxamidsäure oder Brenztraubensäure oder ein Salz davon ist.

6. Ein Reagens, wie in einem der vorhergehenden Ansprüche beansprucht, wobei das Tetrazoliumsalz Tetrazolium-nitroblau ist.

7. Ein Reagens, wie in einem der vorhergehenden Ansprüche beansprucht, das weiterhin Albumin umfaßt.

8. Ein Reagens, wie in irgendeinem der vorhergehenden Ansprüche beansprucht, enthaltend ein nicht-reduzierendes Polyol.

9. Eine diagnostische Reagensausrüstung, umfassend ein Reagens, wie in Anspruch 1 beansprucht, und ein Reagens, wie in Anspruch 2 beansprucht, als Leerreagens, wobei beide Reagentien in lyophilisierter Form vorliegen.

10. Eine Ausrüstung, wie in Anspruch 9 beansprucht, enthaltend ferner Standardproben von Gallensäuren in Humanserum.

**Revendications**

1. Réactif pour la détermination des hydroxystéroïdes dans le sérum, comprenant une hydroxystéroïde-déshydrogénase, une co-enzyme pour celle-ci, à savoir la diaphorase, un sel de tétrazolium et un inhibiteur des hydroxy-acide-déshydrogénases non stéroïdiques, ledit réactif étant substantiellement exempt d'hydroxystéroïdes et d'autres composants sériques.

2. Réactif pour la détermination des hydroxystéroïdes dans la sérum en combinaison avec un réactif selon la revendication 1, comprenant une co-enzyme pour une hydroxystéroïde-déshydrogénase, à savoir la diaphorase, un sel de tétrazolium et inhibiteur des hydroxy-acide-déshydrogénases non

stéroïdiques, ledit réactif étant substantiellement exempt d'hydroxystéroïde-déshydrogénases, d'hydroxystéroïdes et d'autres composants sériques.

3. Réactif selon la revendication 1 ou la revendication 2, sous une forme solide.

4. Réactif selon la revendication 3 sous une forme lyophilisée.

5. Réactif selon l'une quelconque des revendications précédentes, caractérisé en ce que l'inhibiteur est de l'acide oxamique ou de l'acide pyruvique ou un sel de ces acides.

6. Réactif selon l'une quelconque des revendications précédentes, caractérisé en ce que le sel de tétrazolium est le Bleu nitro de tétrazolium.

7. Réactif selon l'une quelconque des revendications précédentes, caractérisé en ce qu'il contient en outre de l'albumine.

8. Réactif selon l'une quelconque des revendications précédentes, caractérisé en ce qu'il contient un polyol non réducteur.

9. Trousse à réactifs, contenant un réactif selon la revendication 1 et un réactif selon la revendication 2 comme réactif blanc, les deux réactifs étant sous forme lyophilisée.

10. Trousse selon la revendication 9, contenant en outre des échantillons normalisés d'acides biliaires dans du sérum humain.